(19) Europäisches Patentamt
European Patent Office
Office européen des brevets

(11) EP 0 719 753 B1

(12) # EUROPÄISCHE PATENTSCHRIFT

(45) Veröffentlichungstag und Bekanntmachung des Hinweises auf die Patenterteilung:
**07.04.1999 Patentblatt 1999/14**

(51) Int Cl.6: **C07C 45/50**, B01J 31/22

(21) Anmeldenummer: **95120578.0**

(22) Anmeldetag: **27.12.1995**

(54) **Verfahren zur Herstellung von Aldehyden**

Process for the preparation of aldehydes

Procédé pour la préparation d'aldéhydes

(84) Benannte Vertragsstaaten:
**AT BE CH DE DK ES FR GB GR IE IT LI LU NL PT SE**

(30) Priorität: **29.12.1994 DE 4447067**

(43) Veröffentlichungstag der Anmeldung:
**03.07.1996 Patentblatt 1996/27**

(73) Patentinhaber: **Celanese GmbH
60439 Frankfurt (DE)**

(72) Erfinder:
- **Herrmann, Wolfgang A., Prof. Dr.
D-85354 Freising (DE)**
- **Elison, Martina
D-80798 München (DE)**
- **Fischer, Jakob
D-85414 Kirchdorf (DE)**
- **Köcher, Christian
D-80805 München (DE)**

(56) Entgegenhaltungen:
- **CHEMICAL ABSTRACTS, vol. 87, no. 25, 1977 Columbus, Ohio, US; abstract no. 200792, TANAKA M ET AL.: "Aldehydes from olefins, carbon monoxide, and hydrogen" Seite 665; Spalte 1; XP002001947 & JP-A-77 083 311 (MITSUI PETROCHEMICAL INDUSTRIES, LTD.)**

EP 0 719 753 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft ein Verfahren zur Herstellung von Aldehyden durch Hydroformylierung von Olefinen in Gegenwart von Komplexverbindungen des Kobalts oder des Rhodiums, die heterocyclische Carbene als Liganden enthalten. Die Reaktion zwischen Olefinen und Kohlenmonoxid und Wasserstoff kann sowohl in homogener als auch in heterogener Phase erfolgen.

Es ist bekannt, durch Umsetzung von Olefinen mit Kohlenmonoxid und Wasserstoff (Hydroformylierung) Aldehyde und Alkohole herzustellen, die ein Kohlenstoffatom mehr als das Ausgangsolefin enthalten. Die Reaktion wird durch Hydridometallcarbonyle, vorzugsweise der Metalle der Gruppen 8, 9, 10 des Periodensystems (entsprechend der IUPAC-Empfehlung von 1985) katalysiert. Neben Kobalt, das als Katalysatormetall ursprünglich und in großem Umfang technisch Verwendung fand, gewinnt in neuerer Zeit Rhodium zunehmend Bedeutung. Im Gegensatz zu Kobalt gestattet Rhodium die Reaktion bei niedrigem Druck durchzuführen. Darüber hinaus werden aus endständigen Olefinen vorzugsweise n-Aldehyde und nur in untergeordnetem Maße iso-Aldehyde gebildet. Schließlich ist auch die Hydrierung der Olefine zu gesättigten Kohlenwasserstoffen in Gegenwart von Rhodium-Katalysatoren deutlich niedriger als bei Anwendung von Kobalt-Katalysatoren. Bei den in der Technik eingeführten Verfahren wird der Rhodium-Katalysator in Form modifizierter Hydridorhodiumcarbonyle eingesetzt, die zusätzliche Liganden, insbesondere tertiäre, organische Phosphine oder Phosphite enthalten. Auch die Kobalt-Katalysatoren werden in Form von Carbonylen angewandt, die zusätzlich Phosphine oder Phosphite als Liganden enthalten, wenngleich diese Variante der Hydroformylierung technisch von geringerer Bedeutung ist als Verfahren, bei denen Rhodium als Katalysator dient.

Zumeist liegen die Liganden, durch die die Aktivität des verwendeten Katalysatormetalls gesteuert wird (daher auch als Steuerliganden bezeichnet), im Überschuß über die zur Bildung der Komplexverbindung erforderlichen Menge vor und stabilisiert dadurch im Sinne des Massenwirkungsgesetzes die Komplexverbindung. Das Katalysatorsystem besteht daher aus Komplexverbindung und freiem Liganden, und der Ligand ist nicht nur für die spezifische katalytische Wirksamkeit, sondern auch für die Stabilität der Komplexverbindung von Bedeutung.

Die Hydroformylierungsreaktion kann sowohl in homogenen als auch in heterogenen Systemen durchgeführt werden. Bei der homogen katalysierten Umsetzung ist der Katalysator im Reaktionsprodukt und einem gegebenenfalls noch vorhandenen Lösungsmittel homogen gelöst. Diese Arbeitsweise hat sich sowohl bei der Verwendung von Kobalt- als auch Rhodium-Katalysatoren gut bewährt. Schwierigkeiten bereitet jedoch die Abtrennung der Reaktionsprodukte und, im Falle der durch Rhodium katalysierten Reaktion, die Wiedergewinnung des Katalysators. Üblicherweise trennt man Produkt und Katalysatorlösung durch Destillation voneinander. Wegen der thermischen Empfindlichkeit der gebildeten Aldehyde und Alkohole kann dieser Weg aber nur bei der Hydroformylierung niedriger Olefine, d.h. Olefine mit bis zu etwa 8 Kohlenstoffatomen im Molekül, beschritten werden.

Die geschilderten Mängel werden bei der durch Rhodium katalysierten Reaktion dadurch vermieden, daß in Wasser lösliche Rhodium-Komplexverbindungen als Katalysatoren eingesetzt werden. Ein entsprechendes Verfahren ist z.B. in der DE-PS 26 27 354 beschrieben. Die Löslichkeit der Rhodium-Komplexverbindungen wird hierbei durch Verwendung sulfonierter Triarylphosphine als Komplexbestandteil erreicht. Die Abtrennung des Katalysators vom Reaktionsprodukt nach Beendigung der Umsetzung erfolgt bei dieser Verfahrens ausgestaltung einfach durch Trennung von wäßriger und organischer Phase, d.h. ohne Destillation und damit ohne zusätzliche thermische Verfahrensschritte. Ein weiteres Merkmal dieser Arbeitsweise ist, daß mit besonders hoher Selektivität aus endständigen Olefinen n-Aldehyde und nur in ganz untergeordnetem Maße iso-Aldehyde gebildet werden. Als Komplexbestandteile wasserlöslicher Rhodium-Komplexverbindungen werden vorzugsweise sulfonierte Triarylphosphine und daneben auch carboxylierte Triarylphosphine eingesetzt.

Organische Phosphine, gleich ob sie in organischen Medien oder in Wasser löslich sind, haben sich als Steuerliganden wegen ihrer stofflichen Vielfalt, ihrer katalytischen Wirksamkeit und ihrer Selektivität in der industriellen Praxis gut bewährt. Dennoch stehen ihrer verbreiteten Anwendung eine Reihe Nachteile entgegen. Unter ihnen ist insbesondere die Oxidationsempfindlichkeit zu nennen, die vor allem in Gegenwart von Metallen und Metallionen auftritt. Daher müssen bei Verwendung von Katalysatoren auf Basis Phosphine enthaltender Komplexverbindungen Maßnahmen zum Ausschluß von Oxidationsmitteln wie Sauerstoff oder Luft ergriffen werden, um die Verluste der häufig nur kostenaufwendig herzustellenden Liganden zu verringern. Eine weitere Eigenschaft, die allen organischen Phosphinen eigen ist und ihre Einsatzmöglichkeit begrenzt, ist die irreversible Spaltung von Phosphor-Kohlenstoff-Bindungen, die z.B. bei der Hydroformylierung oberhalb bestimmter, von der Art des Phosphins abhängiger Temperaturen verstärkt auftritt und zur Deaktivierung des Katalysators und damit die Wirtschaftlichkeit beeinträchtigendem hohen Phosphinverbrauch führt. Schließlich erlauben die herkömmlichen Alkyl- und Arylphosphine ebensowenig wie die gleichfalls als Liganden eingesetzten organischen Phosphite den gesamten Bereich der elektronischen Steuerungsmöglichkeiten bezüglich der katalytisch aktiven Metallzentren abzudecken. Insbesondere fehlen stark nukleophile elektronenreiche Liganden, die beständig gegen Oxidationsmittel sind und eine stabile Bindung mit dem Metall eingehen.

Es bestand daher die Aufgabe, neue Metall-Komplexverbindungen als Katalysatoren für die Hydroformylierungsreaktion zu entwickeln, die die geschilderten Nachteile nicht aufweisen und überdies synthetisch leicht und kostengünstig zugänglich sind. Zudem soll die Konstitution der Steuerliganden auf einfache Weise verändert werden können, so daß es möglich ist, Katalysatoren herzustellen, die individuellen katalytischen Problemen angepaßt werden können.

Diese Aufgabe wird gelöst durch ein Verfahren zur Herstellung von Aldehyden durch Umsetzung von Monoolefinen, Polyolefinen, Cycloolefinen oder Derivaten dieser Verbindungsklassen mit Kohlenmonoxid und Wasserstoff bei Temperaturen von 20 bis 180°C und Drücken von 0,1 bis 30 MPa in Gegenwart von Komplexverbindungen des Kobalts oder des Rhodiums als Katalysator. Es ist dadurch gekennzeichnet, daß die Komplexverbindungen der allgemeinen Formel

$$[L_aM_bX_c]^n(A)_n$$

folgen, in der M für Kobalt oder Rhodium als Zentralatom steht, X an das Zentralatom gebundene ein- oder mehrzähnige, geladene oder ungeladene Liganden bedeuten und L ebenfalls an das Zentralatom M als Liganden gebundene Monocarbene der allgemeinen Formeln

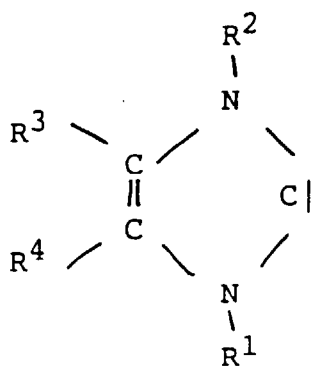

(II)

und

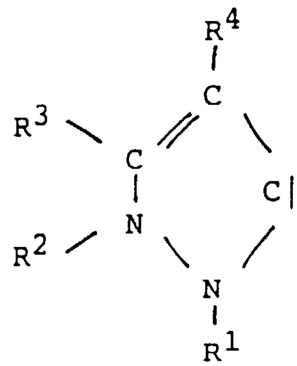

(III)

oder Dicarbene der allgemeinen Formeln

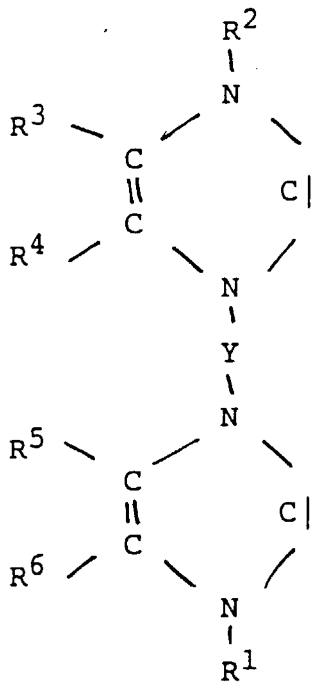

(IV)

und

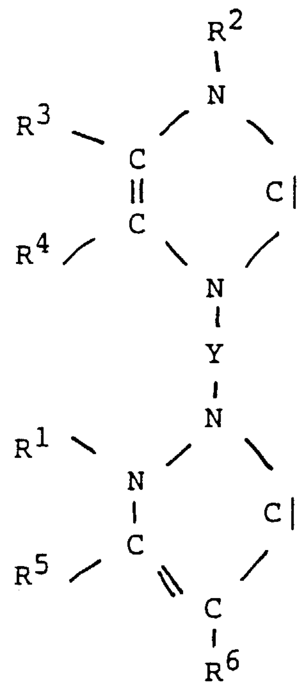

(V)

sind, wobei $R^1$, $R^2$, $R^3$, $R^4$, $R^5$ und $R^6$ gleiche oder verschiedene, geradkettige oder verzweigte, gegebenenfalls sulfonierte Alkylrest mit 1 bis 7 Kohlenstoffatomen, gegebenenfalls sulfonierte aliphatische mono- oder polycyclische Reste mit 5 bis 18 Kohlenstoffatomen, gegebenenfalls sulfonierte Alkenylreste mit 2 bis 5 Kohlenstoffatomen, gegebenenfalls sulfonierte Arylreste mit 6 bis 14 Kohlenstoffatomen oder gegebenenfalls sulfonierte Aralkylreste mit 7 bis 19 Kohlenstoffatomen bedeuten, $R^1$ und/oder $R^2$ auch $-(CH_2)_mPR_2$, mit m = 1, 2, 3 oder 4 und R = Benzyl, Phenyl, substituiertes Phenyl, Naphthyl, ferner $-(CH_2)_mNR'_2$ mit m in der vorstehenden Bedeutung und R' = Alkyl, Vinyl, Allyl, Benzyl und Aryl, sowie $-(CH_2)_mOR''$ mit m in der vorstehenden Bedeutung und R" = Alkyl, insbesondere Methyl, Vinyl, Allyl, Benzyl, Phenyl sein können, $R^3$, $R^4$, $R^5$ und $R^6$ auch für Wasserstoff stehen, $R^3$ und $R^4$ gemeinsam sowie $R^5$ und $R^6$ gemeinsam jeweils auch gleiche oder verschiedene anellierte und gegebenenfalls sulfonierte Reste mit 3 bis 7 Kohlenstoffatomen bedeuten, $R^1$, $R^2$, $R^4$ oder $R^6$ mit Liganden X einen Ring bilden können, Y ein gesättigter oder ungesättigter, geradkettiger oder verzweigter Alkylidenrest mit 1 bis 4 Kohlenstoffatomen oder ein Dialkylsilylen- oder ein Tetraalkyldisilylenrest oder ein 1,3-Phenylenrest ist, A für ein einfach geladenes Anion oder das chemische Äquivalent eines mehrfach geladenen Anions steht, b eine ganze Zahl von 1 bis 3 darstellt, a einer ganzen Zahl von 1 bis 4 • b und c = 0 oder einer ganzen Zahl von 1 bis 4 • b entsprechen und n = 0 oder eine ganze Zahl von 1 bis 6 ist.

Ein- oder mehrzähnige Liganden, die neben den Carbenen in den Komplexverbindungen enthalten sein können und in der allgemeinen Formel (I) durch X wiedergegeben werden, sind Wasserstoff oder das Wasserstoff-Ion, Halogene oder Halogen-Ionen, Pseudohalogenide, Carboxylat-Ionen, Sulfonat-Ionen, Alkylreste mit 1 bis 7 Kohlenstoffatomen, Amidreste, Alkoholatreste, Acetylacetonatreste, Kohlenmonoxid, Stickstoffmonoxid, Nitrile, Isonitrile, Mono- oder Diolefine, Alkine und $\pi$-Aromatenreste. Sind mehrere dieser Liganden im Komplexmolekül enthalten, dann können sie gleich oder verschieden sein.

In den vom Imidazol und vom Pyrazol oder deren Derivaten abgeleiteten Mono- bzw. Dicarbenen entsprechend den Formeln (II), (III), (IV) und (V) stehen $R^1$ bis $R^6$ insbesondere für die Reste Methyl, Isopropyl, tert.-Butyl, Benzyl, Triphenylmethyl, Phenyl, Tolyl, Xylyl, Mesityl und Adamantyl, $R^1$ und $R^2$ sind bevorzugt der Methyl-, der tert.-Butyl-, der Phenyl-, der Benzyl- und der o-Tolylrest, $R^3$ und $R^4$ stehen vorzugsweise für Wasserstoff und die Methylgruppe.

Die Reste $R^3$ und $R^4$ und die Reste $R^5$ und $R^6$ können zusammen mit zwei benachbarten Kohlenstoffatomen des Imidazolringes oder der C-N-Gruppierung im Pyrazolring ein Ringsystem bilden. $R^3$ und $R^4$ bzw. $R^5$ und $R^6$ stehen dann bevorzugt für die Gruppierungen $(CH)_4$, sie führt zur Ausbildung eines anellierten aromatischen Sechsrings, $(CH_2)_4$ und $(CH_2)_5$.

Die durch Y bezeichneten Brückenglieder der Dicarbene gemäß den Formeln (IV) und (V) sind vorzugsweise die Methylen-, Dimethylmethylen-, Diphenylmethylen-, und die Ethylidengruppe. Unter den Silizium enthaltenden Brük-

kengliedern werden die Dimethylsilylen- und die Tetramethyldisilylengruppe bevorzugt.

a ist vorzugsweise 1 oder 2, b vorzugsweise 1; n steht insbesondere für die Zahlen 0 bis 3.

A vertritt bevorzugt Halogenid-, Pseudohalogenid-, Tetraphenylborat-, Tetrafluoroborat-, Hexafluorophosphat- und Carboxylat-Ionen, unter den zuletzt genannten insbesondere das Acetat-Ion, ferner Metallkomplex-Anionen wie z.B. Tetracarbonylcobaltat, Hexafluoroferrat(III), Tetrachloroferrat, Tetrachloroaluminat oder Tetrachloropalladat (II).

Die als Katalysatoren verwendeten Kobalt- und Rhodium-Komplexverbindungen sind auf verschiedenen Wegen zugänglich.

Eine Herstellungsvariante geht von einfachen Verbindungen, d.h. Salzen oder Metallkomplexen (wie den Acetylacetonaten, Metallcarbonylen) jenes Elements aus, das das Zentralatom der Komplexverbindung bildet. Nach einer anderen Variante erhält man die neuen Verbindungen aus Komplexverbindungen durch Ligandenaustausch oder durch Eliminierungs- und/oder Substitutionsreaktionen, zum Beispiel aus gängigen Solvenskomplexen dieser Metallverbindungen. Ferner entstehen die beanspruchten Verbindungen durch einfache Addition des Carbens an die jeweilige Metallkomponente, wobei diese Anlagerung auch unter Aufbrechen einer Brückenstruktur erfolgen kann.

Die Carbene werden entsprechend ihrer Beständigkeit entweder in freier Form als Lösung eingesetzt oder, häufiger, im Reaktionsgemisch aus Verbindungen hergestellt, die unter den Reaktionsbedingungen in Carbene überführt werden können. Die wichtigste Bildungsmethode ist die Deprotonierung von Imidazolium- oder Pyrazoliumsalzen, gegebenenfalls durch Zusatz von Basen wie Metallhydride, Carbonylmetallate, Metallcarboxylate, Metallalkoholate oder Metallamide.

Die Umsetzung der Ausgangsstoffe, d.h. der einfachen Salze bzw. der Komplexverbindungen mit den Carbenen und gegebenenfalls weiteren Liganden erfolgt durch Mischen der Reaktanten in einem Lösungsmittel bei Raumtemperatur oder erhöhter Temperatur. Die Reaktion verläuft mit hoher Geschwindigkeit und ist im wesentlichen oft nach wenigen Minuten beendet. Es empfiehlt sich jedoch zur Vervollständigung der Reaktion Umsetzungszeiten von bis zu mehreren Stunden einzuhalten, insbesondere wenn die Einsatzstoffe im verwendeten Medium nur teilweise gelöst sind, d.h. aus Suspension reagieren.

Zur Herstellung sulfonierte Liganden enthaltender Komplexverbindungen, die wasserlöslich sind, setzt man als Ausgangsstoffe zumindest einen Reaktionspartner ein, dessen Molekül oder Molekülfragment sulfoniert ist.

Zur Isolierung der neuen Komplexverbindungen aus dem Reaktionsmedium hat es sich bewährt, das Lösungsmittel zweckmäßig im Hochvakuum zu entfernen. Das Rohprodukt wird zur Reinigung gewaschen und aus einem geeigneten Lösungsmittel oder Lösungsmittelgemisch, das im Einzelfall durch Vorversuche zu ermitteln ist, umkristallisiert.

Die erfindungsgemäß als Katalysatoren für die Hydroformylierungsreaktion verwendeten Kobalt- und Rhodium-Komplexverbindungen sind in organischen Lösungsmitteln, als Salze auch in Wasser löslich, insbesondere, wenn sie durch Sulfonsäurereste substituierte aliphatische oder aromatische Reste enthalten. Sie sind sehr thermostabil und vielfach auch bei Temperaturen oberhalb 350°C beständig. Bemerkenswert ist überdies, daß sie Oxidationsreaktionen im Gegensatz zu Phosphinen und Phosphiten kaum zugänglich sind. Schließlich neigen die Komplexverbindungen nicht zur Dissoziation. Daher ist es häufig nicht erforderlich, die Liganden zur Steuerung von Katalysatoraktivität und -stabilität im Überschuß über die zur Bildung der Komplexverbindung erforderliche stöchiometrische Menge einzusetzen. Falls es zweckmäßig erscheint, mit einem Ligand-Überschuß zu arbeiten, kann er deutlich niedriger sein als bei Verwendung von Phosphinen oder Phosphiten.

Entsprechend ihrer Löslichkeit werden die katalytisch wirksamen Komplexverbindungen des Kobalts oder Rhodiums entweder im organischen Reaktionsmedium, das üblicherweise aus dem Einsatzolefin, dem Reaktionsprodukt, Reaktionsnebenprodukten und gegebenenfalls einem Lösungsmittel besteht, homogen gelöst eingesetzt. Sie können auch als eigene Phase in wäßriger Lösung in einem heterogenen Reaktionssystem verwendet werden. Als wasserlösliche Katalysatoren kommen insbesondere Komplexverbindungen in Betracht, die durch Sulfonsäurereste substituierte heterogene Carbenliganden enthalten.

Die Kobalt- oder Rhodium-Komplexverbindungen werden in der Regel vor der eigentlichen Reaktion synthetisiert, sie können jedoch auch im Reaktionsgemisch der Hydroformylierung in situ hergestellt werden. Die Gewinnung der Katalysatoren erfolgt in beiden Fällen wie oben beschrieben aus Salzen oder Komplexverbindungen der Metalle Kobalt bzw. Rhodium. Unter der Einwirkung von Synthesegas gehen die ursprünglich eingesetzten Kobalt- oder Rhodium-Komplexverbindungen in den aktiven Hydroformylierungskatalysator über.

Die Konzentration des Metalls (in Form der Komplexverbindung) in der organischen bzw. wäßrigen Katalysatorlösung beträgt, bezogen auf eingesetzte olefinisch ungesättigte Verbindung, $10^{-6}$ bis 1 mol-%, vorzugsweise $10^{-4}$ bis $10^{-1}$ mol-%. Die erforderliche Kobaltkonzentration ist innerhalb der angegebenen Bereiche bis zu einer Zehnerpotenz höher als die erforderliche Rhodiumkonzentration.

Die Umsetzung von olefinisch ungesättigter Verbindung mit Kohlenmonoxid und Wasserstoff erfolgt bei Drükken von etwa 0,1 bis etwa 300 MPa, vorzugsweise 1 bis 15 MPa, wobei Kobaltkatalysatoren höhere Drücke erfordern als Rhodiumkatalysatoren. Die Zusammensetzung des Synthesegases, d.h. das Volumenverhältnis von Kohlenmonoxid und Wasserstoff kann sich über weite Bereiche erstrecken und z.B. zwischen 1 : 10 bis 10 : 1 variiert werden. Im allgemeinen setzt man Gasgemische ein, in denen das Volumenverhältnis von Kohlenmonoxid zu Wasserstoff etwa 1 : 1 ist oder von diesem Wert in der einen oder anderen Richtung nur wenig abweicht.

Die Reaktionstemperatur liegt zwischen etwa 20 und 180°C, bevorzugt werden 80 bis 150°C. Kobaltkatalysatoren erfordern höhere Temperaturen als Katalysatoren auf Basis von Rhodium.

Die Umsetzung der in flüssiger oder gasförmiger Phase vorliegenden Reaktionspartner erfolgt in konventionellen Reaktoren. Der Ablauf der Umsetzung wird maßgeblich dadurch beeinflußt, daß im Fall der homogenen Systeme eine flüssige und eine gasförmige Phase, im Fall der heterogenen Systeme zwei flüssige und eine gasförmige Phase miteinander in innige Berührung gelangen. Es ist daher erforderlich, eine möglichst große Berührungsfläche zwischen den Phasen zu erzeugen. Daher führt man Synthesegas und gegebenenfalls auch Olefin bevorzugt über Verteilungsvorrichtungen der flüssigen Phase zu. Im Fall der mit heterogener Katalysatorphase durchgeführten Hydroformylierungsreaktion empfiehlt es sich, das Reaktionsgemisch intensiv zu rühren. Gegebenenfalls kann der wäßrigen Phase auch ein Lösungsvermittler zugesetzt werden, um die Löslichkeit der olefinischen Verbindung im Katalysator zu verbessern. Die Umsetzung kann absatzweise oder, bevorzugt, kontinuierlich durchgeführt werden.

Das erfindungsgemäße Verfahren ist mit Erfolg auf die Umsetzung von Monoolefinen, Polyolefinen, cyclischen Olefinen und Derivaten dieser ungesättigten Verbindungen anwendbar. Hinsichtlich der Molekülgröße unterliegen die eingesetzten Olefine keiner Beschränkung, die Arbeitsweise hat sich bei Verbindungen mit 2 bis 40 Kohlenstoffatomen bewährt. Die olefinisch ungesättigten Verbindungen können geradkettig oder verzweigt, die Doppelbindungen end- oder innenständig sein. Beispiele für Olefine, die in dem neuen Prozeß verwendet werden können, sind Ethylen, Propylen, Buten-1, Buten-2, Penten-1, 2-Methylbuten-1, Hexen-1, Hexen-2, Hepten-1, Octen-1, Octen-3, 3-Ethylhexen-1, Decen-1, Undecen-3, 4,4-Dimethylnonen-1, Dicyclopentadien, Vinylcyclohexen, Cyclooctadien, Styrol, 2-Vinylnaphthalin. Derivate der genannten Olefinarten, die nach der beanspruchten Arbeitsweise hydroformyliert werden können, sind z.B. Alkohole, Aldehyde, Carbonsäuren, Ester, Nitrile und Halogenverbindungen, Allylalkohol, Acrolein, Methacrolein, Crotonaldehyd, Methylacrylat, Ethylcrotonat, Diethylfumarat, Diethylmaleinat, Acrylnitril. Mit besonderem Erfolg wird das Verfahren zur Hydroformylierung von Olefinen und Olefinderivaten mit 2 bis 20 Kohlenstoffatomen eingesetzt.

Ist in einer der als Katalysator eingesetzten Komplexverbindung einer der Carben-Liganden chiral, d.h. besitzt er kein Symmetrieelement zweiter Art nach Schönflies, so treten in den Hydroformylierungsprodukten optische Induktionen auf, sofern die Einsatzstoffe prochiral sind und der Metallkomplex-Katalysator in optisch reiner Form verwendet wird. Auf diese Weise lassen sich chirale Produkte herstellen.

Im folgenden wird zunächst auf die Herstellung der neuen Katalysatoren eingegangen, die sich anschließenden Beispiele erläutern die Erfindung, beschränken sie aber nicht auf die näher beschriebenen Ausführungsformen.

Beispiel 1: Herstellung von Chloro($\eta^4$-1,5-cyclooctadien)(1,3-dimethylimidazolin-2-yliden)rhodium(I) (Katalysator 1)

a) 1,3-Dimethylimidazolin-2-yliden

8,69 g (38,8 mmol) 1,3-Dimethylimidazolium-iodid werden mit 1,03 g (42,7 mmol) Natriumhydrid und 0,2 g (1,8 mmol) Kalium-tert-butylat in 50 ml Tetrahydrofuran (THF) gelöst und in einem Schlenckrohr mit angeschlossenem Paraffinölrückschlagventil 4 Stunden bei Raumtemperatur gerührt. Die Lösung färbt sich durch das entstehende freie Carben gelb. Das Lösungsmittel wird im Hochvakuum abgezogen und der Rückstand in einer Mikrodestillationsapparatur unter Vakuum destilliert. Man erhält 1,3-Dimethylimidazolin-2-yliden in Form eines gelben Öls. Das Carben wird sofort in 60 ml THF gelöst und bei -30°C aufbewahrt.

b) Chloro($\eta^4$-1,5-cyclooctadien)(1,3-dimethylimidazolin-2-yliden)rhodium(I)

247 mg (0,5 mmol) Di($\mu$-chloro)bis($\eta^4$-1,5-cyclooctadien)dirhodium werden bei Raumtemperatur in 20 ml absolutem THF aufgenommen und mit 192 mg (1 mmol) 1,3-Dimethylimidazolin-2-yliden versetzt. Der sofortige Umsatz ist an einer Farbveränderung von hellgelb nach sattgelb zu erkennen. Man rührt weitere 15 min bei Raumtemperatur, zieht das Lösungsmittel im Hochvakuum ab und reinigt den Rückstand durch Waschen mit 10 ml Diethylether. Das Produkt wird in 10 ml Methylenchlorid aufgenommen und mit 30 ml Pentan vorsichtig überschichtet. Die resultierenden gelben Kristalle werden durch Dekantieren vom Lösungsmittelgemisch befreit und im Hochvakuum getrocknet. Die Verbindung löst sich sehr gut in Chloroform und Methylenchlorid, gut in THF und Toluol, wenig in Diethylether und Pentan mit gelber Farbe. Auch nach mehrstündigem Erhitzen in feuchtem Toluol in einer Sauerstoffatmosphäre findet keine Zersetzung statt. Ausbeute 310 mg (91 %).

5,0 bis 20,0 mg (0,015 bis 0,058 mmol) dieser Verbindung werden jeweils als Katalysator eingesetzt (siehe Tabelle).

Beispiel 2: Herstellung von [($\eta^4$-1,5-Cyclooctadien)bis(1,3-dimethylimidazolin-2-yliden)rhodium(I)]-chlorid (Katalysator 2)

247 mg (0,5 mmol) Di($\mu$-chloro)bis($\eta^4$-1,5-cyclooctadien)dirhodium werden bei Raumtemperatur in 20 ml absolutem THF aufgenommen und mit 279 mg (3 mmol) 1,3-Dimethylimidazolin-2-yliden versetzt. Der sofortige Umsatz ist an einer Farbveränderung von hellgelb nach sattgelb zu erkennen. Man rührt weitere 3 h bei Raumtemperatur, zieht das Lösungsmittel im Hochvakuum ab und reinigt den Rückstand durch Waschen mit 30 ml Diethylether. Das Produkt wird in 10 ml Methylenchlorid aufgenommen und mit 10 ml Pentan vorsichtig überschichtet. Die resultierenden gelben Kristalle werden durch Dekantieren vom Lösungsmittelgemisch befreit und im Hochvakuum getrocknet. Die Verbindung ist gut in Chloroform und Methylenchlorid, mäßig in THF, Wasser und Toluol, nicht in Diethylether und Pentan löslich. Ausbeute 410 mg (93 %).

5,0 bis 20,0 mg (0,011 bis 0,046 mmol) dieser Verbindung werden jeweils als Katalysator eingesetzt (siehe Tabelle).

Beispiel 3: In situ-Herstellung von Katalysator 3

a) 1-Methyl-3-(ethylsulfonsäure-Natriumsalz)imidazoliumbromid

205 mg (2,5 mmol) Methylimidazol werden in Substanz mit 210 mg (1 mmol) 2-Bromethansulfonsäure-Natriumsalz drei Tage bei 70°C gerührt. Nach dem Abkühlen wird der Rückstand zum Entfernen von überschüssigem Methylimidazol dreimal mit 30 ml Diethylether gewaschen. Nach Trocknung im Hochvakuum (70°C, 10 Stunden) verbleibt ein weißer Feststoff, der sehr gut in Wasser, kaum in organischen Solventien (wie THF, Toluol, Pentan) löslich ist. Ausbeute 280 mg (96 %).

b) Katalysator 3

100 mg Rhodium(III)-acetat (0,357 mmol) werden in 25 ml entgastem Wasser gelöst und mit einer Lösung aus 314 mg 1-Methyl-3-(ethyl-2-sulfonsäure-Natriumsalz) (1,07 mmol) in 25 ml entgastem Wasser versetzt. Von dieser Lösung (Rhodiumgehalt 0,0714 mmol je 10 ml Wasser) werden 1,0 bis 5,0 ml (0,00714 bis 0,0357 mmol) als Katalysator eingesetzt (siehe Tabelle), unter Hydroformylierungsbedingungen bildet sich der aktive Hydroformylierungskatalysator.

Beispiel 4: In situ-Herstellung von Katalysator 4

a) 1-(Ethyl-2-sulfonsäure-Natriumsalz)-3-(ethyl-2-sulfonat)imidazoliumbetain

557 mg (8,2 mmol) Imidazol, gelöst in 20 ml Dimethylacetamid, werden mit 1,5 ml (10,25 mmol) Triethylamin und 3,45 g (16,3 mmol) 2-Bromethansulfonsäure-natriumsalz versetzt. Beim Erwärmen auf 120°C klart die ursprüngliche Suspension auf. Nach weiterem Erhitzen auf 160°C beginnt ein weißer Niederschlag auszufallen. Um vollständigen Umsatz zu erzielen, erhitzt man 4 h unter Rückfluß. Nach Abkühlen der Lösung auf Raumtemperatur wird der weiße Niederschlag abfiltriert und 2 mal mit je 20 ml Ethanol und Ether gewaschen.

b) Katalysator 4

100 mg Rhodium(III)-acetat (0,357 mmol) werden in 25 ml entgastem Wasser gelöst und mit einer Lösung aus 328 mg (1,07 mmol) 1-(Ethyl-2-sulfonsäure-Natriumsalz)-3-(ethyl-2-sulfonat)imidazoliumbetain in 25 ml entgastem Wasser versetzt. Aus dieser Lösung (Rhodiumgehalt 0,0714 mmol je 10 ml Wasser) werden 1,0 bis 5,0 ml (0,00714 bis 0,0357 mmol) als Katalysator eingesetzt (siehe Tabelle), unter Hydroformylierungsbedingungen bildet sich der aktive Hydroformylierungskatalysator.

Beispiel 5: In situ-Herstellung von Katalysator 5

a) l-Methyl-3-(butyl-4-sulfonat)imidazoliumbetain

Methylimidazol (8,21 mg, 10 mmol) wird in Substanz mit 1,4-Butansulton (1361 mg, 10 mmol) bei Raumtemperatur 3 Tage lang gerührt. Nach Festwerden der Substanz wäscht man 3 mal mit Toluol und trocknet im Hochvakuum. Der weiße Feststoff löst sich gut in Wasser, weniger gut in organischen Solventien. Ausbeute 2100 mg (96 %).

b) Katalysator 5

100 mg Rhodium(III)-acetat (0,357 mmol) werden in 25 ml entgastem Wasser gelöst und mit einer Lösung aus 234 mg (1,07 mmol) 1-Methyl-3-(butyl-4-sulfonat)imidazoliumbetain in 35 ml entgastem Wasser versetzt. Aus dieser Lösung (Rhodiumgehalt 0,0595 mmol je 10 ml Wasser) werden 1,0 bis 5,0 ml (0,02975 bis 0,00595 mmol) als Katalysator eingesetzt (siehe Tabelle), unter Hydroformylierungsbedingungen bildet sich der aktive Hydroformylierungskatalysator.

Beispiel 6: In situ-Herstellung von Katalysator 6

10,0 mg Rhodium(III)-hexanoat (0,0223 mmol) und 14,6 mg 1,3-Dimethylimidazoliumiodid (0,0669 mmol) werden im Glaseinsatz des Autoklaven eingewogen und mit 25 ml Toluol versetzt. Diese Suspension wird unmittelbar als Katalysator eingesetzt (siehe Tabelle), unter Hydroformylierungsbedingungen bildet sich der aktive Hydroformylierungskatalysator.

Beispiel 7: Hydroformylierung von Olefinen

1000 bis 100.000 Äquivalente eines Olefins werden in einem 250 ml-Rührautoklav (Roth Laborautoklav Typ H 10781 mit Glaseinsatz) in Gegenwart von 5 bis 20 mg der nach den Beispielen 1 und 2 hergestellten und in Toluol gelösten Katalysatoren bzw. 1,0 bis 5,0 ml der wäßrigen Katalysatorlösungen nach den Beispielen 3 bis 5 mit Synthesegas unter Rühren (etwa 150 $min^{-1}$) umgesetzt. Das Synthesegas enthält $H_2$ und CO im Volumenverhältnis 1 : 1, der Gesamtdruck beträgt 10 MPa, der Propenpartialdruck (bei Verwendung von Propen als Olefin) 1,2 MPa. Sobald im Verlaufe der Reaktion der Druck auf etwa 3,0 bis 4,0 MPa absinkt, wird Synthesegas nachgepreßt.
Nach Abkühlen auf Raumtemperatur wird das Produkt mittels Gaschromatographie/Massenspektrometrie analysiert und/oder destillativ gereinigt und charakterisiert.

Anmerkungen zur Tabelle

**[0045]** * Als Produkt konnte ausschließlich 3,4-Dimethylpentanal mittels Gaschromatographie/Massenspektrometrie identifiziert werden.
**Als Produkte konnten durch Gaschromatographie/Massenspektrometrie n-Heptanal (33,8 %) und 2-Methylhexanal (66,2 %) identifiziert werden.

Tabelle - Hydroformylierung von Olefinen

| Nr. | Katalysator | Olefin | Kat./Olefin | Lösungsmittel | n/iso-Verhältnis | Umsatz (%) | Beobachtung |
|---|---|---|---|---|---|---|---|
| 1 | 1 | Propen | 1:100000 | Toluol | 1,0:1,0 | >99(60h) | |
| 2 | 1 | 1-Hexen | 1:100000 | Toluol | 1,0:1,0 | 85(60h) | |
| 3 | 1 | Styrol | 1:2000 | Toluol | 1,16:1,0 | 100(20h) | |
| 4 | 1 | Tetramethylethylen | 1:1000 | Toluol | * | 82(20h) | Eduktisomerisierung |
| 5 | 1 | 2-Hexen | 1:2500 | Toluol | ** | 95(20h) | Eduktisomerisierung |
| 6 | 2 | 1-Hexen | 1:2500 | Toluol | 2,0:1,0 | 85(20h) | Kat. in Toluol schlecht löslich |
| 7 | 2 | Propen | 1:10000 | Wasser | 1,2:1 | 85(20h) | nach Katalyse Kat. in Butyraldehydphase löslich |
| 8 | 3 | Propen | 1:10000 | Wasser | 2,1:1 | 85(20h) | Kat. auch nach Katalyse in wäß. Phase gelöslich; rot |
| 9 | 4 | Propen | 1:10000 | Wasser | 1,9:1 | 92(20h) | Kat. auch nach Katalyse in wäß. Phase gelöst; rot |
| 10 | 5 | Propen | 1:10000 | Wasser | 1,4:1 | 65(20h) | Kat. auch nach Katalyse in wäß. Phase gelöst; rot |
| 11 | 6 | Propen | 1:10000 | Toluol | 1,4:1 | 70(20h) | |

## Patentansprüche

1. Verfahren zur Herstellung von Aldehyden durch Umsetzung von Monoolefinen, Polyolefinen, Cycloolefinen oder Derivaten dieser Verbindungsklassen mit Kohlenmonoxid und Wasserstoff bei Temperaturen von 20 bis 180°C und Drücken von 0,1 bis 30 MPa in Gegenwart von Komplexverbindungen des Kobalts oder des Rhodiums als Katalysator, dadurch gekennzeichnet, daß die Komplexverbindungen der allgemeinen Formel

$$[L_aM_bX_c]^n(A)_n$$

folgen, in der M für Kobalt oder Rhodium als Zentralatom steht, X an das Zentralatom gebundene ein- oder mehrzähnige, geladene oder ungeladene Liganden bedeuten und L ebenfalls an das Zentralatom M als Liganden gebundene Monocarbene der allgemeinen Formeln

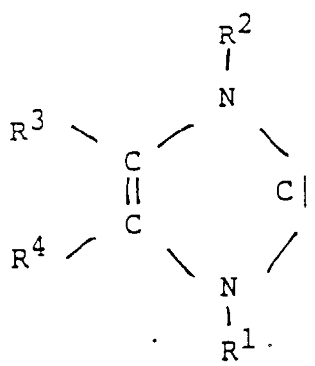

(II)

und

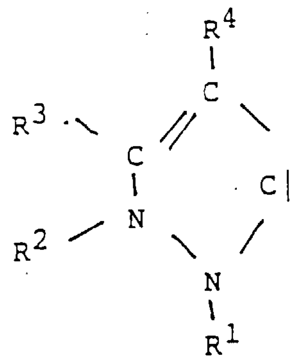

(III)

oder Dicarbene der allgemeinen Formeln

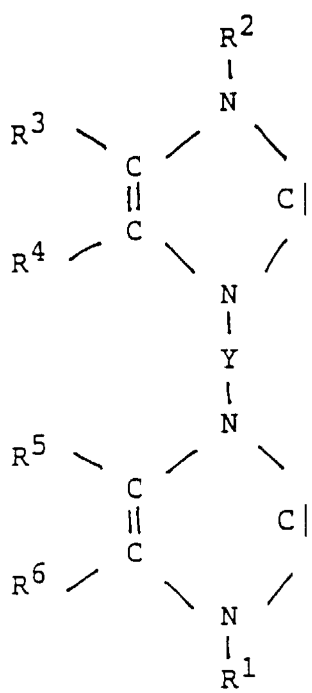

(IV)

und

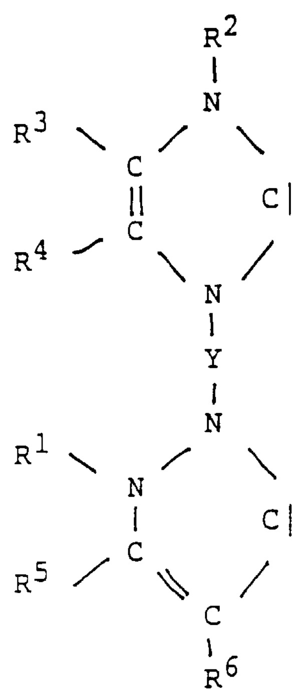

(V)

sind, wobei $R^1$, $R^2$, $R^3$, $R^4$, $R^5$ und $R^6$ gleiche oder verschiedene, geradkettige oder verzweigte, gegebenenfalls sulfonierte Alkylreste mit 1 bis 7 Kohlenstoffatomen, gegebenenfalls sulfonierte aliphatische mono- oder polycyclische Reste mit 5 bis 18 Kohlenstoffatomen, gegebenenfalls sulfonierte Alkenylreste mit 2 bis 5 Kohlenstoffatomen, gegebenenfalls sulfonierte Arylreste mit 6 bis 14 Kohlenstoffatomen oder gegebenenfalls sulfonierte Aralkylreste mit 7 bis 19 Kohlenstoffatomen bedeuten, $R^1$ und/oder $R^2$ auch (CH $-(CH_2)_mPR_2$, mit m = 1, 2, 3 oder 4 und R = Benzyl, Phenyl, substituiertes Phenyl, Naphthyl, ferner $-(CH_2)_mNR'_2$ mit m in der vorstehenden Bedeutung und R' = Alkyl, Vinyl, Allyl, Benzyl und Aryl, sowie $(CH_2)_mOR''$ mit m in der vorstehenden Bedeutung und R" = Alkyl, insbesondere Methyl, Vinyl, Allyl, Benzyl, Phenyl sein können, $R^3$, $R^4$, $R^5$ und $R^6$ auch für Wasserstoff stehen, $R^3$ und $R^4$ gemeinsam sowie $R^5$ und $R^6$ gemeinsam jeweils auch gleiche oder verschiedene anellierte und gegebenenfalls sulfonierte Reste mit 3 bis 7 Kohlenstoffaomen bedeuten, $R^1$, $R^2$, $R^4$ oder $R^6$ mit Liganden X einen Ring bilden können, Y ein gesättigter oder ungesättigter, geradkettiger oder verzweigter Alkylidenrest mit 1 bis 4 Kohlenstoffatomen oder ein Dialkylsilylen- oder ein Tetraalkyldisilylenrest oder ein 1,3-Phenylenrest ist, A für ein einfach gelandenes Anion oder das chemische Äquivalent eines mehrfach geladenen Anions steht, b eine ganze Zahl von 1 bis 3 darstellt, a einer ganzen Zahl von 1 bis 4 • b und c = 0 oder einer ganzen Zahl von 1 bis 4 • b entsprechen und n = 0 oder eine ganze Zahl von 1 bis 6 ist.

**2.** Verfahren nach Anspruch 1 dadurch gekennzeichnet, daß X in der allgemeinen Formel (I) für Wasserstoff, das Wasserstoff-Ion, Halogene, Halogen-Ionen, Pseudohalogenide, Carboxylat-Ionen, Sulfonat-Ionen, Alkylgruppen mit 1 bis 7 Kohlenstoffatomen, Amidreste, Alkoholatreste, Acetylacetonatreste, Kohlenmonoxid, Stickstoffmonoxid, Nitrile, Isonitrile, Mono- oder Diolefine, Alkine und $\pi$-Aromatenreste steht.

**3.** Verfahren nach Anspruch 1 oder 2 dadurch gekennzeichnet, daß in den allgemeinen Formeln (II), (III), (IV) und (V) $R^1$, $R^2$, $R^3$, $R^4$, $R^5$, $R^6$ für die Reste Methyl, Isopropyl, tert.-Butyl, Benzyl, Triphenylmethyl, Phenyl, Tolyl, Xylyl und Mesityl stehen.

**4.** Verfahren nach einem oder mehreren der Ansprüche 1 bis 3 dadurch gekennzeichnet, daß in den allgemeinen Formeln (II), (III), (IV) und (V) $R^3$ und $R^4$ für Wasserstoff und den Methylrest stehen.

**5.** Verfahren nach Anspruch 1 oder 2, dadurch gekennzeichnet, daß in den allgemeinen Formeln (II), (III), (IV) und (V) $R^3$ und $R^4$ gemeinsam und $R^5$ und $R^6$ gemeinsam für die Gruppierungen $(CH)_4$, $(CH_2)_4$, $(CH_2)_5$ stehen.

**6.** Verfahren nach einem oder mehreren der Ansprüche 1 bis 5 dadurch gekennzeichnet, daß in den allgemeinen Formeln (IV) und (V) Y für die Methylen-, die Dimethylmethylen-, die Diphenylmethylen- oder die Ethylidengruppe steht.

**7.** Verfahren nach einem oder mehreren der Ansprüche 1 bis 5 dadurch gekennzeichnet, daß in den allgemeinen Formeln (IV) und (V) Y für die Dimethylsilylen- und die Tetramethyldisilylengruppe steht.

**8.** Verfahren nach einem oder mehreren der Ansprüche 1 bis 7 dadurch gekennzeichnet, daß in der allgemeinen Formel (I) a 1 oder 2 ist.

**9.** Verfahren nach einem oder mehreren der Ansprüche 1 bis 8 dadurch gekennzeichnet, daß in der allgemeinen Formel (I) b 1 ist.

**10.** Verfahren nach einem oder mehreren der Ansprüche 1 bis 9 dadurch gekennzeichnet, daß in der allgemeinen Formel (I) n 0 bis 3 ist.

**11.** Verfahren nach einem oder mehreren der Ansprüche 1 bis 10 dadurch gekennzeichnet, daß in der allgemeinen Formel (I) A für Halogenid- und Pseudohalogenid-Ionen, das Tetraphenylborat-, das Tetrafluoroborat-, das Hexafluorophosphat-, das Acetat-, das Tetracarbonylcobaltat-, das Hexafluoroferrat-, das Tetrachloroferrat-, das Tetrachloroaluminätund das Tetrachloropalladat-Ion steht.

**12.** Verfahren nach einem oder mehreren der Ansprüche 1 bis 11 dadurch gekennzeichnet, daß die Umsetzung der Olefine mit Kohlenmonoxid und Wasserstoff in Gegenwart eines im Reaktionsmedium homogen gelösten Katalysators erfolgt.

**13.** Verfahren nach einem oder mehreren der Ansprüche 1 bis 11, dadurch gekennzeichnet, daß die Umsetzung der Olefine mit Kohlenmonoxid und Wasserstoff in Gegenwart einer wäßrigen Katalysatorlösung erfolgt.

14. Verfahren nach Anspruch 12 oder 13 dadurch gekennzeichnet, daß die Konzentration des Metalls in der organischen Phase oder in der wäßrigen Phase $10^{-6}$ bis 1 mol-%, bezogen auf die eingesetzte olefinisch ungesättigte Verbindung, beträgt.

15. Verfahren nach Anspruch 14 dadurch gekennzeichnet, daß die Konzentration des Metalls in der organischen Phase oder in der wäßrigen Phase $10^{-4}$ bis $10^{-1}$ mol-% beträgt.

16. Verfahren nach einem oder mehreren der Ansprüche 1 bis 15 dadurch gekennzeichnet, daß die Umsetzung bei 80 bis 150°C erfolgt.

## Claims

1. A process for preparing aldehydes by reaction of monoolefins, polyolefins, cycloolefins or derivatives of these classes of compounds with carbon monoxide and hydrogen at temperatures of 20 to 180°C and pressures of 0.1 to 30 MPa in the presence of complex compounds of cobalt or of rhodium as catalyst, characterised in that the complex compounds have the general formula

$$[L_aM_bX_c]^n (A)_n$$

where M is cobalt or rhodium as central atom, X denotes monodentate or multidentate, charged or uncharged ligands bound to the central atom and L likewise stands for ligands bound to the central atom M which are monocarbenes of the general formulae

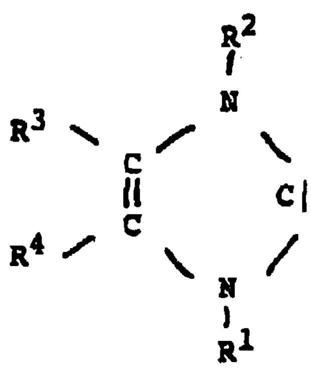

and

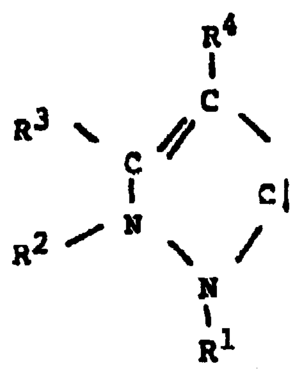

(II) (III)

or dicarbenes of the general formulae

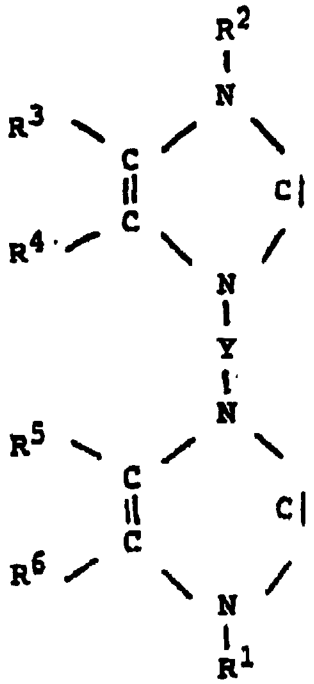

and

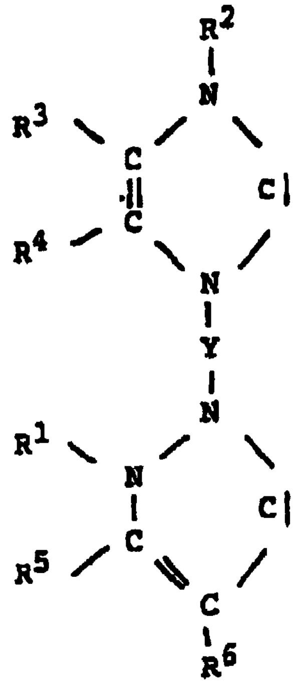

(IV) (V)

where $R^1$, $R^2$, $R^3$, $R^4$, $R^5$ and $R^6$ are identical or different, straight-chain or branched, sulfonated or unsulfonated alkyl radicals having 1 to 7 carbon atoms, sulfonated or unsulfonated aliphatic monocyclic or polycyclic radicals having 5 to 18 carbon atoms, sulfonated or unsulfonated alkenyl radicals having 2 to 5 carbon atoms, sulfonated or unsulfonated aryl radicals having 6 to 14 carbon atoms or sulfonated or unsulfonated aralkyl radicals having 7 to 19 carbon atoms, $R^1$ and/or $R^2$ can also be $(CH_2)_mPR_2$, where m = 1, 2, 3 or 4 and R = benzyl, phenyl, substituted phenyl, naphthyl, furthermore $(CH_2)_mNR'_2$ where m is as defined above and R' = alkyl, vinyl, allyl, benzyl and aryl, and also $(CH_2)_mOR"$ where m is as defined above and R" = alkyl, in particular methyl, vinyl, allyl, benzyl or phenyl; $R^3$, $R^4$, $R^5$ and $R^6$ can also be hydrogen, $R^3$ together with $R^4$ and $R^5$ together with $R^6$ can in each case also be identical or different fused and sulfonated or unsulfonated radicals having 3 to 7 carbon atoms, $R^1$, $R^2$, $R^4$ or $R^6$ with ligand X can form a ring, Y is a saturated or unsaturated, straight-chain or branched alkylidene radical having 1 to 4 carbon atoms or a dialkylsilylene radical or a 1,3-phenylene radical, A is a singly charged anion or the chemical equivalent of a multiply charged anion, b is an integer from 1 to 3, a is an integer from 1 to 4 x b and c = 0 or an integer from 1 to 4 x b and n = 0 or an integer from 1 to 6.

**2.** The process according to claim 1, characterised in that, in the general formula (I), X stands for hydrogen, the hydrogen ion, halogens, halide ions, pseudohalides, carboxylate ions, sulfonate ions, alkyl groups having 1 to 7 carbon atoms, amide radicals, alkoxide radicals, acetylacetonate radicals, carbon monoxide, nitrogen monoxide, nitriles, isonitriles, monoolefins or diolefins, alkynes and $\pi$-aromatic radicals.

**3.** The process according to claim 1 or 2, characterised in that, in the general formulae (II), (III), (IV) and (V), $R^1$, $R^2$, $R^3$, $R^4$, $R^5$, $R^6$ are the radicals methyl, isopropyl, tert.-butyl, benzyl, triphenylmethyl, phenyl, tolyl, xylyl and mesityl.

**4.** The process according to one or more of claims 1 to 3, characterised in that, in the general formulae (II), (III), (IV) and (V), $R^3$ and $R^4$ are hydrogen and the methyl radical.

**5.** The process according to claim 1 or 2, characterised in that, in the general formulae (II), (III), (IV) and (V), $R^3$ together with $R^4$ and $R^5$ together with $R^6$ are the groupings $(CH)_4$, $(CH_2)_4$, $(CH_2)_5$.

**6.** The process according to one or more of claims 1 to 5, characterised in that, in the general formulae (IV) and (V), Y is the methylene group, the dimethylmethylene group, the diphenylmethylene group or the ethylidene group.

**7.** The process according to one or more of claims 1 to 5, characterised in that, in the general formulae (IV) and (V),

Y is the dimethylsilylene group and the tetramethyldisilylene group.

**8.** The process according to one or more of claims 1 to 7, characterised in that, in the general formula (I), a is 1 or 2.

**9.** The process according to one or more of claims 1 to 8, characterised in that, in the general formula (I), b is 1.

**10.** The process according to one or more of claims 1 to 9, characterised in that, in the general formula (I), n is 0 to 3.

**11.** The process according to one or more of claims 1 to 10, characterised in that, in the general formula (I), A is halide or pseudohalide ions, the tetraphenyllorate ion, the tetrafluorolorate ion, the hexafluorophosphate ion, the acetate ion, the tetracarbonylcobaltate ion, the hexafluoroferrate ion, the tetrachloroferrate ion, the tetrachloroaluminate ion and the tetrachloropalladate ion.

**12.** The process according to one or more of claims 1 to 11, characterised in that the reaction of the olefins with carbon monoxide and hydrogen is carried out in the presence of a catalyst homogeneously dissolved in the reaction medium.

**13.** The process according to one or more of claims 1 to 11, characterised in that the reaction of the olefins with carbon monoxide and hydrogen is carried out in the presence of an aqueous catalyst solution.

**14.** The process according to claim 12 to 13, characterised in that the concentration of the metal in the organic phase or in the aqueous phase is $10^{-6}$ to 1 mol %, based on the olefinically unsaturated compound used.

**15.** The process according to claim 14, characterised in that the concentration of the metal in the organic phase or in the aqueous phase is $10^{-4}$ to $10^{-1}$ mol %.

**16.** The process according to one or more of claims 1 to 15, characterised in that the reaction is carried out at 80 to 150°C.

**Revendications**

**1.** Procédé pour la préparation d'aldhéydes par réaction de monooléfines, de polyoléfines, de cyclooléfines ou de dérivés de ces classes de composés avec du monoxyde de carbone et de l'hydrogène à des températures de 20 à 180°C et à des pressions de 0,1 à 30 MPa en présence de complexes du cobalt ou du rhodium comme catalyseur, caractérisé en ce que les complexes suivent la formule générale

$$[L_aM_bX_c]^n(A)_n$$

dans laquelle M représente le cobalt ou le rhodium comme atome central, X représente des coordinats chargés ou non chargés, mono- ou poly-dentés, liés à l'atome central et L représente des monocarbènes des formules générales :

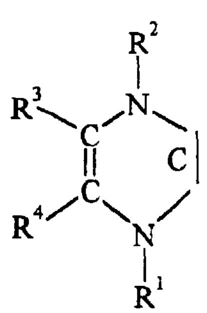

(II)

et

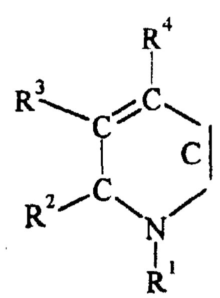

(III)

ou des dicarbènes des formules générales

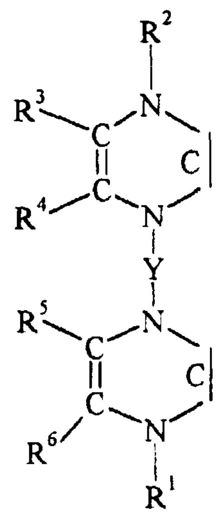

et

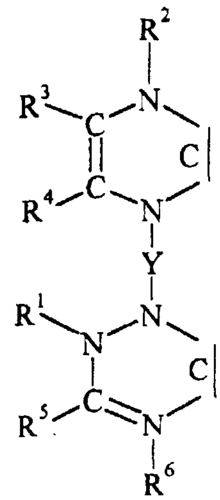

(IV) (V)

également liés comme coordinats à l'atome central M,
$R^1$, $R^2$, $R^3$, $R^4$, $R^5$ et $R^6$ représentant des restes alkyle identiques ou différents, linéaires ou ramifiés, éventuellement sulfonés avec de 1 à 7 atomes de carbone, des restes mono- ou polycycliques, aliphatiques éventuellement sulfonés avec de 5 à 18 atomes de carbone, des restes alcényle éventuellement sulfonés avec de 2 à 5 atomes de carbone, des restes aryle éventuellement sulfonés avec de 6 à 14 atomes de carbone ou des restes aralkyle éventuellement sulfonés avec de 7 à 19 atomes de carbone, $R^1$ et/ou $R^2$ pouvant également être $(CH_2)_m$ $PR_2$ avec m = 1, 2, 3 ou 4 et R = un groupe benzyle, phényle, phényle substitué, naphtyle, en outre $(CH_2)_m$ $NR'_2$ avec m ayant la signification précédente et R' = un groupe alkyle, vinyle, allyle, benzyle et aryle, ainsi que $(CH_2)_mOR''$ avec m ayant la signification précédente et R" = un groupe alkyle, en particulier méthyle, vinyle, allyle, benzyle, phényle, $R^3$, $R^4$, $R^5$ et $R^6$ représentant également l'hydrogène, $R^3$ et $R^4$ ensemble ainsi que $R^5$ et $R^6$ ensemble représentant à chaque fois également des restes identiques ou différents condensés et éventuellement sulfonés avec de 3 à 7 atomes de carbone, $R^1$, $R^2$, $R^4$ ou $R^6$ pouvant former un cycle avec les coordinats X, Y étant un reste alkylidène saturé ou insaturé, linéaire ou ramifié avec de 1 à 4 atomes de carbone ou un reste dialkylsilylène ou tétraalkyldisilylène ou un reste 1,3-phénylène, A représentant un anion chargé une fois ou l'équivalent chimique d'un anion chargé plusieurs fois, b représentant un nombre entier de 1 à 3, a correspondant à un nombre entier de 1 à 4 • b et c = 0 ou un nombre entier de 1 à 4 • b et n = ou étant un nombre entier de 1 à 6.

**2.** Procédé selon la revendication 1, caractérisé en ce que X dans la formule générale (I) représente l'hydrogène, l'ion hydrogène, des halogènes, des ions halogène, des pseudohalogénures, des ions carboxylate, des ions sulfonate, des groupes alkyle avec de 1 à 7 atomes de carbone, des restes amides, des restes alcoolates, des restes acétylacétonate, du monoxyde de carbone, du monoxyde d'azote, des nitriles, des isonitriles, des mono- ou dioléfines, des alcines et des restes β-aromatiques.

**3.** Procédé selon la revendication 1 ou 2, caractérisé en ce que dans les formules générales (II), (III), (IV) et (V) $R^1$, $R^2$, $R^3$, $R^4$, $R^5$ et $R^6$ représentent les restes méthyle, isopropyle, tert.-butyle, triphénylméthyle, phényle, tolyle, xylyle et mésityle.

**4.** Procédé selon l'une ou plusieurs quelconques des revendications 1 à 3, caractérisé en ce que dans les formules générales (II), (III), (IV) et (V) $R^3$ et $R^4$ représentent l'hydrogène et le reste méthyle.

**5.** Procédé selon la revendication 1 ou 2, caractérisé en ce que dans les formules générales (II), (III), (IV) et (V) $R^3$ et $R^4$ ensemble et $R^5$ et $R^6$ ensemble représentent le groupement $(CH)_4$, $(CH_2)_4$, $(CH_2)_5$.

**6.** Procédé selon l'une ou plusieurs quelconques des revendications 1 à 5, caractérisé en ce que dans les formules générales (IV) et (V) Y représente le groupe méthylène, le groupe diméthylméthylène, le groupe diphénylméthylène

ou le groupe éthylidène

7. Procédé selon l'une ou plusieurs quelconques des revendications 1 à 5, caractérisé en ce que dans les formules générales (IV) et (V) Y représente le groupe diméthylsilylène et le groupe tétraméthyldisilylène.

8. Procédé selon l'une ou plusieurs quelconques des revendications 1 à 7, caractérisé en ce que dans la formule générale (I) a est égal à 1 ou à 2.

9. Procédé selon l'une ou plusieurs quelconques des revendications 1 à 8, caractérisé en ce que dans la formule générale (I) b est égal à 1.

10. Procédé selon l'une ou plusieurs quelconques des revendications 1 à 9, caractérisé en ce que dans la formule générale (I) n est compris entre 0 et 3.

11. Procédé selon l'une ou plusieurs quelconques des revendications 1 à 10, caractérisé en ce que dans la formule générale (I) A représente des ions halogénure et pseudohalogènure, l'ion tétraphénylborate, l'ion tétrafluoroborate, l'ion hexafluorophosphate, l'ion acétate, l'ion tétracarbonylcobaltate, l'ion hexafluoroferrate, l'ion tétrachloroferrate, l'ion tétrachloroaluminate et l'ion tétrachloropalladate.

12. Procédé selon l'une ou plusieurs quelconques des revendications 1 à 11, caractérisé en ce que l'on réalise la réaction de l'oléfine avec du monoxyde de carbone et de l'hydrogène en présence d'un catalyseur dissous de manière homogène dans le milieu réactionnel.

13. Procédé selon l'une ou plusieurs quelconques des revendications 1 à 11, caractérisé en ce que l'on réalise la réaction de l'oléfine avec du monoxyde de carbone et de l'hydrogène en présence d'une solution aqueuse de catalyseur.

14. Procédé selon la revendication 12 ou 13, caractérisé en ce que la concentration du métal dans la phase organique ou dans la phase aqueuse est de $10^{-6}$ à 1% en mol, rapportés au composé oléfiniquement insaturé utilisé.

15. Procédé selon la revendication 14, caractérisé en ce que la concentration du métal dans la phase organique ou dans la phase aqueuse est de $10^{-4}$ à $10^{-1}$ % en mol.

16. Procédé selon l'une ou plusieurs quelconques des revendications 1 à 15, caractérisé en ce que l'on réalise la réaction à de 80 à 150°C.